(19) Europäisches Patentamt
European Patent Office
Office européen des brevets

(11) EP 1 713 816 B1

(12) **EUROPÄISCHE PATENTSCHRIFT**

(45) Veröffentlichungstag und Bekanntmachung des Hinweises auf die Patenterteilung:
**01.08.2007 Patentblatt 2007/31**

(21) Anmeldenummer: **05701205.6**

(22) Anmeldetag: **27.01.2005**

(51) Int Cl.:
***C07F 9/00*** *(2006.01)*

(86) Internationale Anmeldenummer:
**PCT/EP2005/000779**

(87) Internationale Veröffentlichungsnummer:
**WO 2005/073241 (11.08.2005 Gazette 2005/32)**

(54) **ABTRENNUNG VON NICKEL(0)-KOMPLEXEN UND PHOSPHORHALTIGEN LIGANDEN AUS NITRILGEMISCHEN**

SEPARATION OF NICKEL(0) COMPLEXES AND PHOSPHORUS-CONTAINING LIGANDS FROM NITRILE MIXTURES

SEPARATION DE COMPLEXES DE NICKEL (0) ET DE LIGANDS PHOSPHORES DANS DES MELANGES DE NITRILE

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **29.01.2004 DE 102004004685**
**15.09.2004 DE 102004045036**

(43) Veröffentlichungstag der Anmeldung:
**25.10.2006 Patentblatt 2006/43**

(73) Patentinhaber: **BASF Aktiengesellschaft**
**67056 Ludwigshafen (DE)**

(72) Erfinder:
- **SCHEIDEL, Jens**
  **69493 Hirschberg (DE)**
- **JUNGKAMP, Tim**
  **B-2950 Kapellen (BE)**
- **BARTSCH, Michael**
  **67433 Neustadt (DE)**
- **HADERLEIN, Gerd**
  **67269 Grünstadt (DE)**
- **BAUMANN, Robert**
  **68159 Mannheim (DE)**
- **LUYKEN, Hermann**
  **67069 Ludwigshafen (DE)**
- **DECKERT, Petra**
  **69245 Bammental (DE)**
- **PFAB, Peter**
  **67433 Neustadt (DE)**
- **SIEGEL, Wolfgang**
  **67117 Limburgerhof (DE)**
- **AECHTNER, Tobias**
  **68163 Mannheim (DE)**

(56) Entgegenhaltungen:
**US-A- 3 773 809**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 713 816 B1

## Beschreibung

Die Erfindung betrifft ein Verfahren zur extraktiven Abtrennung von Nickel(0)-Komplexen mit phosphorhaltigen Liganden und/oder freien phosphorhaltigen Liganden, aus einem Reaktionsaustrag einer Hydrocyanierung von ungesättigten Mononitrilen zu Dinitrilen, durch Extraktion mittels eines Kohlenwasserstoffs, wobei bei einer Temperatur T (in °C) eine Phasentrennung des Kohlenwasserstoffs und des Reaktionsaustrages in zwei Phasen erfolgt,
dadurch gekennzeichnet, dass im Reaktionsaustrag der Hydrocyanierung der Gehalt an Nickel(0)-Komplexen mit phosphorhaltigen Liganden und/oder freien phosphorhaltigen Liganden, abhängig von der Temperatur T mindestens y Gew.-% beträgt, und unabhängig von der Temperatur T maximal 60 Gew.-% beträgt, wobei der Zahlenwert des Mindestgehalts y durch die Gleichung

$$y = 0{,}5 \cdot T + 20$$

gegeben ist und T in die Gleichung als dimensionsloser Zahlenwert einzusetzen ist.

Für Hydrocyanierungen von ungesättigten Mononitrilen sind Nickelkomplexe von Phosphorliganden geeignete Katalysatoren. So wird beispielsweise Adipodinitril, ein wichtiges Intermediat in der Nylonproduktion, durch zweifache Hydrocyanierung von 1,3-Butadien hergestellt. Dabei wird in einer ersten Hydrocyanierung 1,3-Butadien mit Cyanwasserstoff in Gegenwart von Nickel(0), das mit Phosphorliganden stabilisiert ist, zu 3-Pentennitril umgesetzt. In einer zweiten Hydrocyanierung wird anschließend 3-Pentennitril mit Cyanwasserstoff zu Adipodinitril ebenfalls an einem Nikkel-Katalysator, allerdings gegebenenfalls unter Zusatz einer Lewis-Säure und evtl. eines Promotors, umgesetzt. Nickel (0) oder Ni(0) bedeutet Nickel in der Oxidationsstufe 0.

Um die Wirtschaftlichkeit der Hydrocyanierung zu erhöhen, wird üblicherweise der Nickelkatalysator abgetrennt und zurückgeführt (Katalysatorkreislauf). Da das Katalysatorsystem in der zweiten Hydrocyanierung, das eine Mischung aus Komplex und freiem Liganden darstellt, thermisch wenig belastbar ist, kann die Abtrennung des hochsiedenden Adipodinitrils vom Katalysatorsystem nicht destillativ erfolgen. Daher wird die Trennung im Allgemeinen extraktiv mit Cyclohexan oder Methylcyclohexan als Extraktionsmittel durchgeführt. Das Katalysatorsystem verbleibt dabei - idealerweise vollständig, unter realen Bedingungen zumindest teilweise - in der leichteren Cyclohexan- oder Methylcyclohexanphase, während die schwerere Phase polarer ist und rohes Adipodinitril und ggf. die Lewis-Säure enthält. Das Extraktionsmittel wird nach der Phasentrennung in der Regel destillativ unter vermindertem Druck abgetrennt. Der Siededruck des Extraktionsmittels ist dabei deutlich höher als der des Adipodinitrils.

In den US-PS 3,773,809 und 5,932,772 wird die Extraktion des Katalysatorkomplexes und der Liganden mit Paraffinen und Cycloparaffinen, beispielsweise Cyclohexan, Heptan und Octan, oder Alkylaromaten, beschrieben.

Aus der US-PS 4,339,395 ist ein Verfahren zur extraktiven Aufarbeitung von Reaktionsausträgen von Hydrocyanierungen für Katalysatorsysteme mit monodentalen Liganden und einem Triarylboran als Promotor bekannt, bei dem eine geringe Menge Ammoniak zudosiert wird, um Mulmbildung zu vermeiden.

Die WO 2004/062765 beschreibt die extraktive Abtrennung eines Nickel-Diphosphit-Katalysators aus einem Gemisch von Mono- und Dinitrilen mit Alkanen oder Cycloalkanen als Extraktionsmittel, wobei das Gemisch mit einer Lewis-Base, z.B. Organoaminen oder Ammoniak, behandelt wird.

Aus der US-PS 5,847,191 ist ein Verfahren zur extraktiven Aufarbeitung von Reaktionsausträgen von Hydrocyanierungen bekannt, wobei die Chelatliganden $C_9$- bis $C_{40}$-Alkylreste tragen.

Die US-PS 4,990,645 beschreibt, dass die Extraktionsfähigkeit des Nickelkomplexes und des freien Liganden verbessert werden kann, wenn der in der Reaktion gebildete Feststoff $Ni(CN)_2$ vor der Extraktion in einem Dekanter abgetrennt wird. Dazu wird zuvor ein Teil des Pentennitrils abgedampft, um die Löslichkeit des Katalysators und des $Ni(CN)_2$ zu verringern.

Um eine Phasentrennung zwischen Cyclohexan- bzw. Methylcyclohexanphase und der rohen Adipodinitrilhaltigen Phase zu erzielen, musste bisher ein Mindestumsatz des 3-Pentennitrils erzielt werden. So wird in der US-PS 3,773,809 als Bedingung für die Phasentrennung bei Verwendung von Cyclohexan als Extraktionsmittel ein Mindestumsatz des 3-Pentennitrils von 60 % gefordert, so dass das Verhältnis zwischen 3-Pentennitril und Adipodinitril unter 0,65 beträgt. Wenn dieses Verhältnis durch Umsetzung von 3-Pentennitril nicht erreicht wird, muss entweder 3-Pentennitril vorverdampft oder Adipodinitril beigemischt werden, um auf ein Verhältnis von unter 0,65 zu kommen. Problematisch bei diesem Mindestumsatz von 3-Pentennitril ist, dass mit einem höheren Umsatzgrad an 3-Pentennitril eine schlechtere Selektivität von Adipodinitril bezüglich 3-Pentennitril und Cyanwasserstoff verbunden ist. Darüber hinaus führt ein Mindestumsatz des 3-Pentennitrils von 60 % zu einer geringeren Standzeit des Katalysatorsystems.

Der vorliegenden Erfindung lag daher die Aufgabe zugrunde, den zuvor genannten Nachteilen abzuhelfen, also ein Verfahren zur extraktiven Abtrennung von Nickel(0)-Komplexen mit phosphorhaltigen Liganden und/oder freien phosphorhaltigen Liganden aus einem Reaktionsaustrag einer Hydrocyanierung von ungesättigten Mononitrilen zu Dinitrilen bereitzustellen, das die zuvor beschriebenen Nachteile der bekannten Verfahren vermeidet. Insbesondere soll es bei dem erfindungsgemäßen Verfahren möglich sein, die extraktive Abtrennung von Nickel(0)-Komplexen mit phosphorhaltigen Liganden und/oder freien phosphorhaltigen Liganden aus einem Reaktionsaustrag einer Hydrocyanierung durchzuführen, bei dem ein geringerer Umsatz an ungesättigtem Mononitril gefahren werden muss und bei dem eine Vorverdampfung des ungesättigten Mononitrils oder eine Beimengung des Dinitrils nicht zwingend notwendig ist.

Demgemäß wurde das eingangs genannte Verfahren gefunden. Bevorzugte Ausführungsformen der Erfindung sind den Unteransprüchen zu entnehmen.

In einer besonders bevorzugten Ausführungsform wird das erfindungsgemäße Verfahren bei der Herstellung von Adipodinitril verwendet. Somit ist das erfindungsgemäße Verfahren vorzugsweise für 3-Pentennitril als Mononitril und Adipodinitril als Dinitril bestimmt. Ebenso bevorzugt wird der Reaktionsaustrag der Hydrocyanierung durch Umsetzung von 3-Pentennitril mit Cyanwasserstoff in Gegenwart mindestens eines Nickel(0)-Komplexes mit phosphorhaltigen Liganden, gegebenenfalls in Gegenwart mindestens einer Lewis-Säure (z.B. als Promotor), erhalten.

Verfahrensprinzip

Das erfindungsgemäße Verfahren eignet sich zur extraktiven Abtrennung von Ni(0)-Komplexen, die phosphorhaltige Liganden und/oder freie phosphorhaltige Liganden enthalten, aus einem Reaktionsaustrag, der bei einer Hydrocyanierung von ungesättigten Mononitrilen zu Dinitrilen anfällt. Diese Komplexe werden weiter unten beschrieben.

Der Reaktionsaustrag wird mittels eines Kohlenwasserstoffs extrahiert; dabei tritt bei einer Temperatur T (in °C) eine Phasentrennung des Kohlenwasserstoffs und des Reaktionsaustrages in zwei Phasen ein. In der Regel bildet sich eine erste Phase, die gegenüber dem Reaktionsaustrag an den genannten Ni(0)-Komplexen bzw. Liganden angereichert ist, und eine zweite Phase, die gegenüber dem Reaktionsaustrag an Dinitrilen angereichert ist. Zumeist ist die erste Phase die leichtere Phase, also die Oberphase, und die zweite Phase die schwerere Phase, also die Unterphase.

Erfindungsgemäß beträgt im Reaktionsaustrag der Hydrocyanierung der maximale Gehalt an Nickel(0)-Komplexen mit phosphorhaltigen und/oder freien Liganden, 60 Gew.-%. Dieser Maximalgehalt ist unabhängig von der Temperatur T. Der Mindestgehalt an den genannten Ni(0)-Komplexen bzw. Liganden ist abhängig von T und beträgt y Gew.-%, wobei der Zahlenwert des Mindestgehalts y durch die Gleichung

$$y = 0,5 \cdot T + 20$$

gegeben ist und T als dimensionsloser Zahlenwert eingesetzt wird. Beträgt beispielsweise die Temperatur T der Phasentrennung 50°C, so ist y = 0,5.50 + 20 = 45; der Mindestgehalt beträgt bei T = 50°C demnach 45 Gew.-%.

Die Extraktion weist bevorzugt je nach Phasenverhältnis einen Extraktionskoeffizienten - definiert als Verhältnis aus dem Massengehalt an den genannten Nickel(0)-Komplexen bzw. Liganden in der Oberphase zum Massengehalt an den genannten Nickel(0)-Komplexen bzw. Liganden in der Unterphase - für jede theoretische Extraktionsstufe, von 0,1 bis 10, besonders bevorzugt 0,8 bis 5, auf. Dabei ist die Extraktionswirkung gemessen am Extraktionskoeffizienten für den freien Liganden gleich gut oder besser, bevorzugt besser als für den Nickel(0)-Komplex.

Die Oberphase enthält nach der Phasentrennung vorzugsweise zwischen 50 und 99 Gew.%, besonders bevorzugt zwischen 60 und 97 Gew.-%, insbesondere zwischen 80 und 95 Gew.-%, des zur Extraktion eingesetzten Kohlenwasserstoffes.

Die Lewis-Säure, die gegebenenfalls (nämlich bei der eingangs erwähnten zweiten Hydrocyanierung) im Zulaufstrom der Extraktion enthalten ist, verbleibt vorzugsweise größtenteils und besonders bevorzugt vollständig in der Unterphase. Hier bedeutet vollständig, dass die Restkonzentration der Lewis-Säure in der Oberphase vorzugsweise kleiner als 1 Gew.-%, besonders bevorzugt kleiner als 0,5 Gew.%, insbesondere kleiner als 500 ppm by weight ist.

Kohlenwasserstoff

Der Kohlenwasserstoff ist das Extraktionsmittel. Er weist bevorzugt einen Siedepunkt von mindestens 30, besonders bevorzugt mindestens 60, insbesondere mindestens 90°C, und bevorzugt höchstens 140, besonders bevorzugt höchstens 135, insbesondere höchstens 130°C auf, jeweils bei einem Druck von $10^5$ Pa absolut.

Besonders bevorzugt kann ein Kohlenwasserstoff, wobei im Sinne der vorliegenden Erfindung hierunter ein einzelner Kohlenwasserstoff, wie auch ein Gemisch solcher Kohlenwasserstoffe verstanden wird, zur Abtrennung, insbesondere durch Extraktion, von Adipodinitril aus einer Mischung, enthaltend Adipodinitril und den Ni(0) enthaltenden Katalysator, eingesetzt werden, der einen Siedepunkt im Bereich zwischen 90°C und 140°C aufweist. Aus der nach der Abtrennung gemäß diesem Verfahren erhaltenen Mischung kann der Katalysator - ggf. unter Zusatz eines geeigneten Lösungsmittels, das höhersiedend ist als der Kohlenwasserstoff K (z.B. Pentennitril) - vorteilhaft durch destillative Abtrennung des Kohlenwasserstoffs erhalten werden, wobei der Einsatz eines Kohlenwasserstoffs mit einem Siedepunkt in dem genannten Bereich eine besonders wirtschaftliche und technisch einfache Abtrennung durch die Möglichkeit der Kondensierung des abdestillierten Kohlenwasserstoffs mit Flusswasser gestattet.

Geeignete Kohlenwasserstoffe sind beispielsweise in US 3,773,809, Spalte 3, Zeile 50-62, beschrieben. Vorzugsweise kommt ein Kohlenwasserstoff, ausgewählt aus Cyclohexan, Methylcyclohexan, Cycloheptan, n-Hexan, n-Heptan, isomeren Heptanen, n-Octan, iso-Octan, isomeren Octanen wie 2,2,4-Trimethylpentan, cis- und trans-Decalin oder deren Gemische, insbesondere aus Cyclohexan, Methylcyclohexan, n-Heptan, isomeren Heptanen, n-Octan, isomeren Octanen wie 2,2,4-Trimethylpentan, oder deren Gemische, in Betracht. Besonders bevorzugt verwendet man Cyclohexan, Methylcyclohexan, n-Heptan oder n-Octan.

Ganz besonders bevorzugt sind n-Heptan oder n-Octan. Bei diesen Kohlenwasserstoffen ist die unerwünschte Mulmbildung besonders gering. Unter Mulm wird ein Bereich unvollständiger Phasentrennung zwischen Ober- und Unterphase verstanden, meist ein flüssig/flüssig-Gemisch, in dem auch Feststoffe dispergiert sein können. Übermäßige Mulmbildung ist unerwünscht, da sie die Extraktion behindert und u.U. die Extraktionsvorrichtung vom Mulm geflutet werden kann, wodurch sie ihre Trennaufgabe nicht mehr erfüllen kann.

Der verwendete Kohlenwasserstoff ist vorzugsweise wasserfrei, wobei wasserfrei einen Wassergehalt von unter 100, vorzugsweise unter 50, insbesondere unter 10 ppm by weight bedeutet. Der Kohlenwasserstoff kann durch geeignete, dem Fachmann bekannte Verfahren getrocknet werden, beispielsweise durch Adsorption oder Azeotropdestillation. Die Trocknung kann in einem dem erfindungsgemäßen Verfahren vorgeschalteten Schritt erfolgen.

Ausgestaltung der Extraktion

Die Extraktion der Nickel(0)-Komplexe bzw. Liganden aus dem Reaktionsaustrag kann in jeder geeigneten, dem Fachmann bekannten Vorrichtung durchgeführt werden, bevorzugt in Gegenstrom-Extraktionskolonnen, Mixer-Settler-Kaskaden oder Kombinationen von Mixer-Settler-Kaskaden mit Kolonnen. Besonders bevorzugt ist die Verwendung von Gegenstrom-Extraktionskolonnen, die insbesondere mit Blechpackungen als dispergierenden Elemente ausgestattet sind. In einer weiteren besonders bevorzugten Ausführungsform wird die Extraktion im Gegenstrom in einer kompartimentierten, gerührten Extraktionskolonne ausgeführt.

Betreffend die Dispergierrichtung wird in einer bevorzugten Ausführungsform des Verfahrens der Kohlenwasserstoff als kontinuierliche Phase und der Reaktionsaustrag der Hydrocyanierung als disperse Phase eingesetzt. Dies verkürzt in der Regel die Phasentrennzeit und vermindert die Mulmbildung. Jedoch ist auch die umgekehrte Dispergierrichtung, also Reaktionsaustrag als kontinuierliche und Kohlenwasserstoff als disperse Phase, möglich. Letzteres gilt insbesondere dann, wenn die Mulmbildung durch vorherige Feststoffabtrennung (siehe weiter unten), höhere Temperatur bei der Extraktion bzw. Phasentrennung oder Verwendung eines geeigneten Kohlenwasserstoffs reduziert oder vollständig unterdrückt wird. Üblicherweise wählt man die für die Trennleistung der Extraktionsvorrichtung günstigere Dispergierrichtung.

In der Extraktion wird ein Phasenverhältnis von vorzugsweise 0,1 bis 10, besonders bevorzugt 0,4 bis 2,5, insbesondere 0,75 bis 1,5, jeweils berechnet als Verhältnis von Masse des zugeführten Kohlenwasserstoffs zu Masse der zu extrahierenden Mischung, verwendet.

Der absolute Druck während der Extraktion beträgt vorzugsweise 10 kPa bis 1 MPa, besonders bevorzugt 50 kPa bis 0,5 MPa, insbesondere 75 kPa bis 0,25 MPa (absolut).

Die Extraktion wird vorzugsweise bei einer Temperatur von -15 bis 120, insbesondere 20 bis 100 und besonders bevorzugt 30 bis 80°C durchgeführt. Es wurde gefunden, dass bei höherer Temperatur der Extraktion die Mulmbildung geringer ist.

In einer besonders bevorzugten Ausführungsform wird die Extraktion mit einem Temperaturprofil betrieben. Insbesondere arbeitet man in diesem Fall bei einer Extraktionstemperatur von mindestens 60, bevorzugt 60 bis 95 und besonders bevorzugt mindestens 70°C.

Das Temperaturprofil ist bevorzugt derart ausgestaltet, dass in demjenigen Bereich der Extraktion, worin der Gehalt an Nickei(0)-Kompiexen mit phosphorhaltigen Liganden und/oder freien phosphorhaltigen Liganden höher ist als im anderen Bereich, die Temperatur niedriger ist als im anderen Bereich. Auf diese Weise werden die temperaturlabilen Ni(0)-Komplexe thermisch weniger belastet und ihr Zerfall vermindert.

Verwendet man zur Extraktion beispielsweise eine Extraktionskolonne und wendet ein Temperaturprofil an, so wird am Kolonnenkopf die niedrigste und am Kolonnensumpf die höchste Temperatur eingestellt. Die Temperaturdifferenz zwischen Kolonnenkopf und -sumpf kann z.B. 0 bis 30, bevorzugt 10 bis 30 und insbesondere 20 bis 30°C betragen.

Ausgestaltung der Phasentrennung

Die Phasentrennung kann räumlich und zeitlich je nach apparativer Ausgestaltung auch als letzter Teil der

Extraktion betrachtet werden. Zur Phasentrennung kann üblicherweise ein weiter Druck-, Konzentrations- und Temperaturbereich gewählt werden, wobei die für die jeweilige Zusammensetzung der Reaktionsmischung optimalen Parameter leicht durch wenige einfache Vorversuche ermittelt werden können.

Die Temperatur T bei der Phasentrennung beträgt üblicherweise mindestens 0, vorzugsweise mindestens 10, besonders bevorzugt mindestens 20°C. Üblicherweise beträgt sie höchstens 120, vorzugsweise höchstens 100, besonders bevorzugt höchstens 95°C. Beispielsweise führt man die Phasentrennung bei 0 bis 100, bevorzugt 60 bis 95°C durch. Es wurde gefunden, dass bei höherer Temperatur der Phasentrennung die Mulmbildung geringer ist.

Der Druck bei der Phasentrennung liegt in der Regel bei mindestens 1 kPa, vorzugsweise mindestens 10 kPa, besonders bevorzugt 20 kPa. In der Regel beträgt er höchstens 2 MPa, vorzugsweise höchstens 1 MPa, besonders bevorzugt höchstens 0,5 MPa absolut.

Die Phasentrennzeit, also die Zeitspanne von der Vermischung des Reaktionsaustrages mit dem Kohlenwasserstoff (Extraktionsmittel) bis zur Ausbildung einer einheitlichen Oberphase und einer einheitlichen Unterphase, kann in weiten Grenzen variieren. Die Phasentrennzeit beträgt in der Regel 0,1 bis 60, bevorzugt 1 bis 30 und insbesondere 2 bis 10 min. Bei großtechnischer Durchführung des erfindungsgemäßen Verfahrens ist üblicherweise eine Phasentrennzeit von maximal 15, insbesondere maximal 10 min technisch und ökonomisch sinnvoll.

Es wurde gefunden, dass sich die Phasentrennzeit insbesondere bei Verwendung langkettiger aliphatischer Alkane wie n-Heptan oder n-Octan in vorteilhafter Weise vermindert.

Die Phasentrennung kann in einer oder mehreren dem Fachmann für solche Phasentrennungen bekannten Vorrichtungen durchgeführt werden. In einer vorteilhaften Ausführungsform kann man die Phasentrennung in der Extraktionsvorrichtung durchführen, beispielsweise in einer oder mehreren Mixer-Settler-Kombinationen oder durch Ausstattung einer Extraktionskolonne mit einer Beruhigungszone.

Bei der Phasentrennung erhält man zwei flüssige Phasen, von denen eine Phase einen höheren Anteil an dem Nickel(0)-Komplex mit phosphorhaltigen Liganden und/oder freien phosphorhaltigen Liganden, bezogen auf das Gesamtgewicht dieser Phase, aufweist als die andere Phase oder anderen Phasen.

In einer bevorzugten Ausführungsform des Verfahrens wird bei einer Temperatur der Phasentrennung von 20°C ein Adipodinitril-Gehalt des Austragsstroms aus der Hydrocyanierung von größer 30 Gew.-% eingestellt, wobei der Gehalt an Nickel(0)-Komplexen bzw. Liganden kleiner 60 Gew.-%, vorzugsweise kleiner 50 Gew.-%, besonders bevorzugt kleiner 40 Gew.-% ist.

In einer weiteren bevorzugten Ausführungsform des Verfahrens wird bei einer Temperatur der Phasentrennung von 40°C ein Adipodinitril-Gehalt des Austragsstroms aus der Hydrocyanierung von größer 40 Gew.-% eingestellt, wobei der Gehalt an Nickel(0)-Komplexen bzw. Liganden kleiner 60 Gew.-%, vorzugsweise kleiner 50 Gew.-%, besonders bevorzugt kleiner 40 Gew.-% ist.

In einer weiteren bevorzugten Ausführungsform des erfindungsgemäßen Verfahrens wird bei einer Temperatur der Phasentrennung von 60°C ein Adipodinitril-Gehalt des Austragsstroms aus der Hydrocyanierung von größer 50 Gew.-% eingestellt, wobei der Gehalt an Nickel(0)-Komplexen bzw. Liganden kleiner 50 Gew.-%, vorzugsweise kleiner 40 Gew.-% ist.

Optionale Behandlung mit Ammoniak oder Amin

In einer bevorzugten Ausführungsform des erfindungsgemäßen Verfahrens behandelt man den Reaktionsaustrag der Hydrocyanierung vor oder während der Extraktion, mit Ammoniak oder einem primären, sekundären oder tertiären aromatischen oder aliphatischen Amin. Aromatisch schließt alkylaromatisch, und aliphatisch schließt cycloaliphatisch ein.

Es wurde gefunden, dass sich durch diese Ammoniak- bzw. Aminbehandlung der Gehalt an Nickel(0)-Komplex bzw. Ligand in der zweiten, mit Dinitrilen angereicherten Phase (meist Unterphase) vermindern lässt, d.h. die Verteilung des Ni(0)-Komplexes bzw. Liganden auf die beiden Phasen wird zugunsten der ersten Phase (Oberphase) verschoben. Die Ammoniak- bzw. Aminbehandlung verbessert die Katalysatoranreicherung in der Oberphase; dies bedeutet geringere Katalysatorverluste im Katalysatorkreislauf und verbessert die Wirtschaftlichkeit der Hydrocyanierung.

Demnach geht in dieser Ausführungsform der Extraktion eine Behandlung des Reaktionsaustrags mit Ammoniak bzw. einem Amin voraus oder erfolgt während der Extraktion. Dabei ist die Behandlung während der Extraktion weniger bevorzugt.

Als Amine verwendet man Monomamine, Diamine, Triamine oder höherfuktionelle Amine (Polyamine). Die Monoamine weisen üblicherweise Alkylreste, Arylreste oder Arylalkylreste mit 1 bis 30 C-Atomen auf; geeignete Monoamine sind z.B. primäre Amine, z.B. Monoalkylamine, sekundäre oder tertiäre Amine, z.B. Dialkylamine. Geeignete primäre Monoamine sind beispielsweise Butylamin, Cyclohexylamin, 2-Methylcyclohexylamin, 3-Methylcyclohexylamin, 4-Methylcyclohexylamin, Benzylamin, Tetrahydrofurfurylamin und Furfurylamin. Als sekundäre Monoamine kommen z.B. Diethylamin, Dibutylamin, Di-n-propylamin und N-Methylbenzylamin in Betracht. Als tertiäre Amine eignen sich beispielsweise Trialkylamine mit $C_{1-10}$-Alkylresten, wie Trimethylamin, Triethylamin oder Tributylamin.

Als Diamine eignen sich z.B. solche der Formel $R^1$-NH-$R^2$-NH-$R^3$, worin $R^1$, $R^2$ und $R^3$ unabhängig voneinander Wasserstoff oder einen Alkylrest, Arylrest oder Arylalkylrest mit 1 bis 20 C-Atomen bedeuten. Der Alkylrest kann linear oder insbesondere für $R^2$ auch cyclisch sein. Geeignete Diamine sind beispielsweise Ethylendiamin, die Propylendiamine (1,2-Diaminopropan and 1,3-Diaminopropan), N-Methyl-ethylendiamin, Piperazin, Tetramethylendiamin (1,4-Diaminobutan), N,N'-Dimethylethylendiamin, N-Ethylethylendiamin, 1,5-Diaminopentan, 1,3-Diamino-2,2-diethylpropan, 1,3-Bis (methylamino)propan, Hexamethylendiamin (1,6-Diaminohexan), 1,5-Diamino-2-methylpentan, 3-(Propylamino)-propylamin, N,N'-Bis-(3-aminopropyl)-piperazin, N,N'-Bis-(3-aminopropyl)-piperazin und Isophorondiamin (IPDA).

Als Triamine, Tetramine bzw. höherfunktionelle Amine eignen sich z.B. Tris(2-aminoethyl)amin, Tris(2-aminopropyl)amin, Diethylentriamin (DETA), Triethylentetramin (TE-TA), Tetraethylenpentamin (TEPA), Isopropylentriamin, Dipropylentriamin und N,N'-bis(3-aminopropyl-ethylendiamin). Aminobenzylamine und Aminohydrazide mit 2 oder mehr Aminogruppen sind ebenfalls geeignet.

Naturgemäß kann man auch Mischungen von Ammoniak mit einem oder mehreren Aminen, oder Mischungen mehrerer Amine verwenden.

Bevorzugt verwendet man Ammoniak oder aliphatische Amine, insbesondere Trialkylamine mit 1 bis 10 C-Atomen im Alkylrest, z.B. Trimethylamin, Triethylamin oder Tributylamin, sowie Diamine wie Ethylendiamin, Hexamehtylendiamin oder 1,5-Diamino-2-methylpentan.

Besonders bevorzugt ist Ammoniak allein, d.h. besonders bevorzugt verwendet man neben Ammoniak kein Amin. Wasserfreier Ammoniak ist ganz besonders bevorzugt; dabei bedeutet wasserfrei einen Wassergehalt unter 1 Gew.-%, bevorzugt unter 1000 und insbesondere unter 100 ppm by weight.

Das Molverhältnis von Amin zu Ammoniak kann in weiten Grenzen variiert werden, liegt in der Regel bei 10000 : 1 bis 1 : 10000.

Die Menge des eingesetzten Ammoniaks bzw. Amins richtet sich u.a. nach Art und Menge des Nickel(0)-Katalysators und/oder der Liganden, und - sofern mitverwendet - nach Art und Menge der Lewis-Säure, die bei der Hydrocyanierung als Promotor eingesetzt wird. Üblicherweise beträgt das Molverhältnis von Ammoniak bzw. Amin zu Lewis-Säure mindestens 1: 1. Die Obergrenze dieses Molverhältnisses ist in der Regel unkritisch und beträgt beispielsweise 100 : 1; der Überschuss an Ammoniak bzw. Amin sollte jedoch nicht so groß sein, dass sich der Ni(0)-Komplex bzw. dessen Liganden zersetzen. Bevorzugt beträgt das Molverhältnis Ammoniak bzw. Amin zu Lewis-Säure 1 : 1 bis 10 : 1, besonders bevorzugt 1,5 : 1 bis 5 : 1, und insbesondere etwa 2,0 : 1. Sofern man eine Mischung aus Ammoniak und Amin verwendet, gelten diese Molverhältnisse für die Summe aus Ammoniak und Amin.

Die Temperatur bei der Behandlung mit Ammoniak bzw. Amin ist üblicherweise nicht kritisch und beträgt beispielsweise 10 bis 140, bevorzugt 20 bis 100 und insbesondere 20 bis 90°C. Auch der Druck ist in der Regel nicht kritisch.

Der Ammoniak bzw. das Amin kann dem Reaktionsaustrag gasförmig, flüssig (unter Druck stehend) oder gelöst in einem Lösungsmittel zugegeben werden. Als Lösungsmittel eignen sich z.B. Nitrile, insbesondere solche, die bei der Hydrocyanierung vorliegen, und weiterhin aliphatische, cycloaliphatische oder aromatische Kohlenwasserstoffe, wie man sie bei dem erfindungsgemäßen Verfahren als Extraktionsmittel verwendet, beispielsweise Cyclohexan, Methylcyclohexan, n-Heptan odem-Octan.

Die Ammoniak- bzw. Aminzugabe erfolgt in üblichen Vorrichtungen, beispielsweise solchen zur Gaseinleitung oder in Flüssigkeitsmischern. Der dabei in vielen Fällen ausfallende Feststoff kann entweder im Reaktionsaustrag verbleiben, d.h. der Extraktion wird eine Suspension zugeführt, oder abgetrennt werden wie nachfolgend beschrieben.

Optionale Abtrennung der Feststoffe

In einer bevorzugten Ausführungsform trennt man vor der Extraktion die im Reaktionsaustrag enthaltenen Feststoffe zumindest teilweise ab. Dadurch lässt sich in vielen Fällen die Extraktionsleistung des erfindungsgemäßen Verfahrens weiter verbessern. Es wird vermutet, dass ein hoher Feststoffgehalt den Stoffübergang während der Extraktion behindert, was größere und damit teurere Extraktionsvorrichtungen erforderlich macht. Außerdem wurde gefunden, dass eine Feststoffabtrennung vor der Extraktion die unerwünschte Mulmbildung oftmals deutlich vermindert oder vollständig unterdrückt.

Bevorzugt wird die Feststoffabtrennung derart ausgestaltet, dass Feststoffpartikel mit einem hydraulischen Durchmesser größer 5 $\mu$m, insbesondere größer 1 $\mu$m und besonders bevorzugt größer 100 nm, abgetrennt werden.

Zur Feststoffabtrennung kann man übliche Verfahren verwenden, beispielsweise Filtration, Querstromfiltration, Zentrifugation, Sedimentation, Klassierung oder bevorzugt Dekantieren, wozu gängige Vorrichtungen wie Filter, Zentrifugen bzw. Dekanter verwendet werden können.

Temperatur und Druck bei der Feststoffabtrennung sind üblicherweise nicht kritisch. Beispielsweise kann man in den zuvor genannten Temperatur- bzw. Druckbereichen arbeiten.

Die Feststoffabtrennung kann vor, während oder nach der - optionalen - Behandlung des Reaktionsaustrages mit Ammoniak bzw. Amin erfolgen. Dabei ist die Abtrennung während oder nach der Ammoniak- bzw. Aminbehandlung

bevorzugt, und danach besonders bevorzugt.

Sofern man die Feststoffe während oder nach der Ammoniak- bzw. Aminbehandlung abtrennt, handelt es sich bei den Feststoffen zumeist um im Reaktionsaustrag schwerlösliche Verbindungen von Ammoniak bzw. Amin mit der verwendeten Lewis-Säure bzw. dem Promotor. Verwendet man beispielsweise $ZnCl_2$, so fällt bei der Ammoniakbehandlung im Wesentlichen schwerlösliches $ZnCl_2 \bullet 2\ NH_3$ aus.

Sofern man die Feststoffe vor der Ammoniak- bzw. Aminbehandlung abtrennt oder falls gar keine Behandlung mit Ammoniak oder Amin erfolgt, handelt es sich bei den Feststoffen in der Regel um Nickelverbindungen der Oxidationsstufe +II, beispielsweise Nickel(II)cyanid oder ähnliche cyanidhaltige Nickel(II)verbindungen.

Nickel(0)-Komplexe und Liganden

Bei den Ni(0)-Komplexen, die phosphorhaltige Liganden und/oder freie phosphorhaltige Liganden enthalten, handelt es sich bevorzugt um homogen gelöste Nickel(0)-Komplexe.

Die phosphorhaltigen Liganden der Nickel(0)-Komplexe und die freien phosphorhaltigen Liganden, die erfindungsgemäß durch Extraktion abgetrennt werden, sind vorzugsweise ausgewählt aus mono- oder bidentaten Phosphinen, Phosphiten, Phosphiniten und Phosphoniten.

Diese phosphorhaltigen Liganden weisen vorzugsweise die Formel I auf:

$$P(X^1R^1)\ (X^2R^2)\ (X^3R^3) \qquad (I)$$

Unter Verbindung I wird im Sinne der vorliegenden Erfindung eine einzelne Verbindung oder ein Gemisch verschiedener Verbindungen der vorgenannten Formel verstanden.

Erfindungsgemäß sind $X^1$, $X^2$, $X^3$ unabhängig voneinander Sauerstoff oder Einzelbindung. Falls alle der Gruppen $X^1$, $X^2$ und $X^3$ für Einzelbindungen stehen, so stellt Verbindung I ein Phosphin der Formel $P(R^1R^2R^3)$ mit den für $R^1$, $R^2$ und $R^3$ in dieser Beschreibung genannten Bedeutungen dar.

Falls zwei der Gruppen $X^1$, $X^2$ und $X^3$ für Einzelbindungen stehen und eine für Sauerstoff, so stellt Verbindung I ein Phosphinit der Formel $P(OR^1)(R^2)(R^3)$ oder $P(R^1)(OR^2)(R^3)$ oder $P(R^1)(R^2)(OR^3)$ mit den für $R^1$, $R^2$ und $R^3$ weiter unten genannten Bedeutungen dar.

Falls eine der Gruppen $X^1$, $X^2$ und $X^3$ für eine Einzelbindung steht und zwei für Sauerstoff, so stellt Verbindung I ein Phosphonit der Formel $P(OR^1)(OR^2)(R^3)$ oder $P(R^1)(OR^2)(OR^3)$ oder $P(OR^1)(R^2)(OR^3)$ mit den für $R^1$, $R^2$ und $R^3$ in dieser Beschreibung genannten Bedeutungen dar.

In einer bevorzugten Ausführungsform sollten alle der Gruppen $X^1$, $X^2$ und $X^3$ für Sauerstoff stehen, so dass Verbindung I vorteilhaft ein Phosphit der Formel $P(OR^1)(OR^2)(OR^3)$ mit den für $R^1$, $R^2$ und $R^3$ weiter unten genannten Bedeutungen darstellt.

Erfindungsgemäß stehen $R^1$, $R^2$, $R^3$ unabhängig voneinander für gleiche oder unterschiedliche organische Reste. Als $R^1$, $R^2$ und $R^3$ kommen unabhängig voneinander Alkylreste, vorzugsweise mit 1 bis 10 Kohlenstoffatomen, wie Methyl, Ethyl, n-Propyl, i-Propyl, n-Butyl, i-Butyl, s-Butyl, t-Butyl, Aryl-Gruppen, wie Phenyl, o-Tolyl, m-Tolyl, p-Tolyl, 1-Naphthyl, 2-Naphthyl, oder Hydrocarbyl, vorzugsweise mit 1 bis 20 Kohlenstoffatomen, wie 1,1'-Biphenol, 1,1'-Binaphthol in Betracht. Die Gruppen $R^1$, $R^2$ und $R^3$ können miteinander direkt, also nicht allein über das zentrale Phosphor-Atom, verbunden sein. Vorzugsweise sind die Gruppen $R^1$, $R^2$ und $R^3$ nicht miteinander direkt verbunden.

In einer bevorzugten Ausführungsform kommen als Gruppen $R^1$, $R^2$ und $R^3$ Reste ausgewählt aus der Gruppe bestehend aus Phenyl, o-Tolyl, m-Tolyl und p-Tolyl in Betracht. In einer besonders bevorzugten Ausführungsform sollten dabei maximal zwei der Gruppen $R^1$, $R^2$ und $R^3$ Phenyl-Gruppen sein.

In einer anderen bevorzugten Ausführungsform sollten dabei maximal zwei der Gruppen $R^1$, $R^2$ und $R^3$ o-Tolyl-Gruppen sein.

Als besonders bevorzugte Verbindungen I können solche der Formel Ia

$$(\text{o-Tolyl-O-})_w\ (\text{m-Tolyl-O-})_x\ (\text{p-Tolyl-O-})_y\ (\text{Phenyl-O-})_z\ P \qquad (Ia)$$

eingesetzt werden, wobei w, x, y und z eine natürliche Zahl bedeuten, und folgende Bedingungen gelten: $w + x + y + z = 3$ und $w, z \leq 2$.

Solche Verbindungen Ia sind z.B. (p-Tolyl-O-)(Phenyl-O-)$_2$P, (m-Tolyl-O-)(Phenyl-O-)$_2$P, (o-Tolyl-O-) (Phenyl-O-)$_2$P, (p-Tolyl-O-)$_2$(Phenyl-O-)P, (m-Tolyl-O-)$_2$(Phenyl-O-)P, (o-Tolyl-O-)$_2$(Phenyl-O-)P, (m-Tolyl-O-)(p-Tolyl-O)(Phenyl-O-)P, (o-Tolyl-O-)(p-Tolyl-O-)(Phenyl-O-)P, (o-Tolyl-O-)(m-Tolyl-O-)(Phenyl-O-)P, (p-Tolyl-O-)$_3$P, (m-Tolyl-O-)(p-Tolyl-O-)$_2$P, (o-Tolyl-O-)(p-Tolyl-O-)$_2$P, (m-Tolyl-O-)$_2$(p-Toluyl-O-)P, (o-Tolyl-O-)$_2$(p-Tolyl-O-)P, (o-Tolyl-O-)(m-Tolyl-O-)(p-Tolyl-O)P, (m-Tolyl-O-)$_3$P, (o-Tolyl-O-)(m-Tolyl-O-)$_2$P (o-Tolyl-O-)$_2$(m-Tolyl-O-)P, oder Gemische solcher Verbindungen.

Gemische enthaltend (m-Tolyl-O-)$_3$P, (m -Tolyl-O-)$_2$(p-Tolyl-O-)P, (m-Tolyl-O-)(p-Tolyl-O-)$_2$P und (p-Tolyl-

$O\text{-})_3P$ kann man beispielsweise durch Umsetzung eines Gemisches enthaltend m-Kresol und p-Kresol, insbesondere im Molverhältnis 2 : 1, wie es bei der destillativen Aufarbeitung von Erdöl anfällt, mit einem Phosphortrihalogenid, wie Phosphortrichlorid, erhalten.

In einer anderen, ebenfalls bevorzugten Ausführungsform kommen als phosphorhaltige Liganden die in der DE-A 199 53 058 näher beschriebenen Phosphite der Formel Ib in Betracht:

$$P(O\text{-}R^1)_x\ (O\text{-}R^2)_y\ (O\text{-}R^3)_z\ (O\text{-}R^4)_p \qquad \text{(Ib)}$$

mit

$R^1$: aromatischer Rest mit einem $C_1$-$C_{18}$-Alkylsubstituenten in o-Stellung zu dem Sauerstoffatom, das das Phosphoratom mit dem aromatischen System verbindet, oder mit einem aromatischen Substituenten in o-Stellung zu dem Sauerstoffatom, das das Phosphoratom mit dem aromatischen System verbindet, oder mit einem in o-Stellung zu dem Sauerstoffatom, das das Phosphoratom mit dem aromatischen System verbindet, anellierten aromatischen System,

$R^2$: aromatischer Rest mit einem $C_1$-$C_{18}$-Alkylsubstituenten in m-Stellung zu dem Sauerstoffatom, das das Phosphoratom mit dem aromatischen System verbindet, oder mit einem aromatischen Substituenten in m-Stellung zu dem Sauerstoffatom, das das Phosphoratom mit dem aromatischen System verbindet, oder mit einem in m-Stellung zu dem Sauerstoffatom, das das Phosphoratom mit dem aromatischen System verbindet, anellierten aromatischen System, wobei der aromatische Rest in o-Stellung zu dem Sauerstoffatom, das das Phosphoratom mit dem aromatischen System verbindet, ein Wasserstoffatom trägt,

$R^3$: aromatischer Rest mit einem $C_1$-$C_{18}$-Alkylsubstituenten in p-Stellung zu dem Sauerstoffatom, das das Phosphoratom mit dem aromatischen System verbindet, oder mit einem aromatischen Substituenten in p-Stellung zu dem Sauerstoffatom, das das Phosphoratom mit dem aromatischen System verbindet, wobei der aromatische Rest in o-Stellung zu dem Sauerstoffatom, das das Phosphoratom mit dem aromatischen System verbindet, ein Wasserstoffatom trägt,

$R^4$: aromatischer Rest, der in o-, m- und p-Stellung zu dem Sauerstoffatom, das das Phosphoratom mit dem aromatischen System verbindet, andere als die für $R^1$, $R^2$ und $R^3$ definierten Substituenten trägt, wobei der aromatische Rest in o-Stellung zu dem Sauerstoffatom, das das Phosphoratom mit dem aromatischen System verbindet, ein Wasserstoffatom trägt,

x: 1 oder 2,

y, z, p: unabhängig voneinander 0, 1 oder 2 mit der Maßgabe, dass x+y+z+p = 3.

Bevorzugte Phosphite der Formel Ib sind der DE-A 199 53 058 zu entnehmen. Als Rest $R^1$ kommen vorteilhaft o-Tolyl-, o-Ethyl-phenyl-, o-n-Propyl-phenyl-, o-Isopropylphenyl-, o-n-Butyl-phenyl-, o-sek-Buty1-phenyl-, o-tert-Butyl-phenyl-, (o-Phenyl)-Phenyl- oder 1-Naphthyl- Gruppen in Betracht.

Als Rest $R^2$ sind m-Tolyl-, m-Ethyl-phenyl-, m-n-Propyl-phenyl-, m-Isopropyl-phenyl-, m-n-Butyl-phenyl-, m-sek-Butyl-phenyl-, m-tert-Butyl-phenyl-, (m-Phenyl)-Phenyl- oder 2-Naphthyl- Gruppen bevorzugt.

Als Rest $R^3$ kommen vorteilhaft p-Tolyl-, p-Ethyl-phenyl-, p-n-Propyl-phenyl-, p-Isopropyl-phenyl-, p-n-Butyl-phenyl-, p-sek-Butyl-phenyl-, p-tert-Butyl-phenyl- oder (p-Phenyl)-Phenyl-Gruppen in Betracht.

Rest $R^4$ ist bevorzugt Phenyl. Vorzugsweise ist p gleich null. Für die Indizes x, y, z und p in Verbindung Ib ergeben sich folgende Möglichkeiten:

| x | y | z | p |
|---|---|---|---|
| 1 | 0 | 0 | 2 |
| 1 | 0 | 1 | 1 |
| 1 | 1 | 0 | 11 |
| 2 | 0 | 0 | 1 |
| 1 | 0 | 2 | 0 |

(fortgesetzt)

| x | y | z | p |
|---|---|---|---|
| 1 | 1 | 1 | 0 |
| 1 | 2 | 0 | 0 |
| 2 | 0 | 1 | 0 |
| 2 | 1 | 0 | 0 |

Bevorzugte Phosphite der Formel Ib sind solche, in denen p gleich null ist sowie $R^1$, $R^2$ und $R^3$ unabhängig voneinander ausgewählt sind aus o-Isopropyl-phenyl, m-Tolyl und p-Tolyl, und $R^4$ Phenyl ist.

Besonders bevorzugte Phosphite der Formel Ib sind solche, in denen $R^1$ der o-Isopropyl-phenyl-Rest, $R^2$ der m-Tolylrest und $R^3$ der p-Tolylrest ist mit den in der vorstehenden Tabelle genannten Indizes; außerdem solche, in denen $R^1$ der o-Tolylrest, $R^2$ der m-Tolylrest und $R^3$ der p-Tolylrest ist mit den in der Tabelle genannten Indizes; weiterhin solche, in denen $R^1$ der 1-Naphthylrest, $R^2$ der m-Tolylrest und $R^3$ der p-Tolylrest ist mit den in der Tabelle genannten Indizes; außerdem solche, in denen $R^1$ der o-Tolylrest, $R^2$ der 2-Naphthylrest und $R^3$ der p-Tolylrest ist mit den in der Tabelle genannten Indizes; und schließlich solche, in denen $R^1$ der o-Isopropyl-phenyl-Rest, $R^2$ der 2-Naphthylrest und $R^3$ der p-Tolylrest ist mit den in der Tabelle genannten Indizes; sowie Gemische dieser Phosphite.

Phosphite der Formel Ib können erhalten werden, indem man

a) ein Phosphortrihalogenid mit einem Alkohol ausgewählt aus der Gruppe bestehend aus $R^1OH$, $R^2OH$, $R^3OH$ und $R^4OH$ oder deren Gemische umsetzt unter Erhalt eines Dihalogenophosphorigsäuremonoesters,

b) den genannten Dihalogenophosphorigsäuremonoester mit einem Alkohol ausgewählt aus der Gruppe bestehend aus $R^1OH$, $R^2OH$, $R^3OH$ und $R^4OH$ oder deren Gemische umsetzt unter Erhalt eines Monohalogenophosphorigsäurediesters und

c) den genannten Monohalogenophosphorigsäurediester mit einem Alkohol ausgewählt aus der Gruppe bestehend aus $R^1OH$, $R^2OH$, $R^3OH$ und $R^4OH$ oder deren Gemische umsetzt unter Erhalt eines Phosphits der Formel Ib.

Die Umsetzung kann in drei getrennten Schritten durchgeführt werden. Ebenso können zwei der drei Schritte kombiniert werden, also a) mit b) oder b) mit c). Alternativ können alle der Schritte a), b) und c) miteinander kombiniert werden.

Dabei kann man geeignete Parameter und Mengen der Alkohole ausgewählt aus der Gruppe bestehend aus $R^1OH$, $R^2OH$, $R^3OH$ und $R^4OH$ oder deren Gemische durch einige einfache Vorversuche leicht ermitteln.

Als Phosphortrihalogenid kommen grundsätzlich alle Phosphortrihalogenide, vorzugsweise solche, in denen als Halogenid Cl, Br, I, insbesondere Cl, eingesetzt wird, sowie deren Gemische in Betracht. Es können auch Gemische verschiedener gleich oder unterschiedlich halogensubstituierter Phosphine als Phosphortrihalogenid eingesetzt werden. Besonders bevorzugt ist $PCl_3$. Weitere Einzelheiten zu den Reaktionsbedingungen bei der Herstellung der Phosphite I b und zur Aufarbeitung sind der DE-A 199 53 058 zu entnehmen.

Die Phosphite Ib können auch in Form eines Gemisches verschiedener Phosphite Ib als Ligand verwendet werden. Ein solches Gemisch kann beispielsweise bei der Herstellung der Phosphite Ib anfallen.

Es ist allerdings bevorzugt, dass der phosphorhaltige Ligand mehrzähnig, insbesondere zweizähnig ist. Daher weist der verwendete Ligand vorzugsweise die Formel II

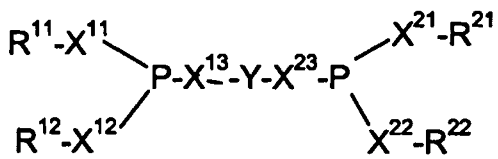

**(II)**

auf, worin bedeuten

$X^{11}$, $X^{12}$, $X^{13}$, $X^{21}$, $X^{22}$, $X^{23}$ unabhängig voneinander Sauerstoff oder Einzelbindung

$R^{11}$, $R^{12}$ unabhängig voneinander gleiche oder unterschiedliche, einzelne oder verbrückte organische Reste

$R^{21}$, $R^{22}$ unabhängig voneinander gleiche oder unterschiedliche, einzelne oder verbrückte organische Reste,

Y Brückengruppe

Unter Verbindung II wird im Sinne der vorliegenden Erfindung eine einzelne Verbindung oder ein Gemisch verschiedener Verbindungen der vorgenannten Formel verstanden.

In einer bevorzugten Ausführungsform können $X^{11}$, $X^{12}$, $X^{13}$, $X^{21}$, $X^{22}$, $X^{23}$ Sauerstoff darstellen. In einem solchen Fall ist die Brückengruppe Y mit Phosphit-Gruppen verknüpft.

In einer anderen bevorzugten Ausführungsform können $X^{11}$ und $X^{12}$ Sauerstoff und $X^{13}$ eine Einzelbindung oder $X^{11}$ und $X^{13}$ Sauerstoff und $X^{12}$ eine Einzelbindung darstellen, so dass das mit $X^{11}$, $X^{12}$ und $X^{13}$ umgebene Phosphoratom Zentralatom eines Phosphonits ist. In einem solchen Fall können $X^{21}$, $X^{22}$ und $X^{23}$ Sauerstoff oder $X^{21}$ und $X^{22}$ Sauerstoff und $X^{23}$ eine Einzelbindung oder $X^{21}$ und $X^{23}$ Sauerstoff und $X^{22}$ eine Einzelbindung oder $X^{23}$ Sauerstoff und $X^{21}$ und $X^{22}$ eine Einzelbindung oder $X^{21}$ Sauerstoff und $X^{22}$ und $X^{23}$ eine Einzelbindung oder $X^{21}$, $X^{22}$ und $X^{23}$ eine Einzelbindung darstellen, so dass das mit $X^{21}$, $X^{22}$ und $X^{23}$ umgebene Phosphoratom Zentralatom eines Phosphits, Phosphonits, Phosphinits oder Phosphins, vorzugsweise eines Phosphonits, sein kann.

In einer anderen bevorzugten Ausführungsform können $X^{13}$ Sauerstoff und $X^{11}$ und $X^{12}$ eine Einzelbindung oder $X^{11}$ Sauerstoff und $X^{12}$ und $X^{13}$ eine Einzelbindung darstellen, so dass das mit $X^{11}$, $X^{12}$ und $X^{13}$ umgebene Phosphoratom Zentralatom eines Phosphonits ist. In einem solchen Fall können $X^{21}$, $X^{22}$ und $X^{23}$ Sauerstoff oder $X^{23}$ Sauerstoff und $X^{21}$ und $X^{22}$ eine Einzelbindung oder $X^{21}$ Sauerstoff und $X^{22}$ und $X^{23}$ eine Einzelbindung oder $X^{21}$, $X^{22}$ und $X^{23}$ eine Einzelbindung darstellen, so dass das mit $X^{21}$, $X^{22}$ und $X^{23}$ umgebene Phosphoratom Zentralatom eines Phosphits, Phosphinits oder Phosphins, vorzugsweise eines Phosphinits, sein kann.

In einer anderen bevorzugten Ausführungsform können $X^{11}$, $X^{12}$ und $X^{13}$ eine Einzelbindung darstellen, so dass das mit $X^{11}$, $X^{12}$ und $X^{13}$ umgebene Phosphoratom Zentralatom eines Phosphins ist. In einem solchen Fall können $X^{21}$, $X^{22}$ und $X^{23}$ Sauerstoff oder $X^{21}$, $X^{22}$ und $X^{23}$ eine Einzelbindung darstellen, so dass das mit $X^{21}$, $X^{22}$ und $X^{23}$ umgebene Phosphoratom Zentralatom eines Phosphits oder Phosphins, vorzugsweise eines Phosphins, sein kann.

Als Brückengruppe Y kommen vorzugsweise substituierte, beispielsweise mit $C_1$-$C_4$-Alkyl, Halogen, wie Fluor, Chlor, Brom, halogeniertem Alkyl, wie Trifluormethyl, Aryl, wie Phenyl, oder unsubstituierte Arylgruppen in Betracht, vorzugsweise solche mit 6 bis 20 Kohlenstoffatomen im aromatischen System, insbesondere Pyrocatechol, Bis(phenol) oder Bis(naphthol).

Die Reste $R^{11}$ und $R^{12}$ können unabhängig voneinander gleiche oder unterschiedliche organische Reste darstellen. Vorteilhaft kommen als Reste $R^{11}$ und $R^{12}$ Arylreste, vorzugsweise solche mit 6 bis 10 Kohlenstoffatomen, in Betracht, die unsubstituiert oder einfach oder mehrfach substituiert sein können, insbesondere durch $C_1$-$C_4$-Alkyl, Halogen, wie Fluor, Chlor, Brom, halogeniertem Alkyl, wie Trifluormethyl, Aryl, wie Phenyl, oder unsubstituierte Arylgruppen.

Die Reste $R^{21}$ und $R^{22}$ können unabhängig voneinander gleiche oder unterscheidliche organische Reste darstellen. Vorteilhaft kommen als Reste $R^{21}$ und $R^{22}$ Arylreste, vorzugsweise solche mit 6 bis 10 Kohlenstoffatomen, in Betracht, die unsubstituiert oder einfach oder mehrfach substituiert sein können, insbesondere durch $C_1$-$C_4$-Alkyl, Halogen, wie Fluor, Chlor, Brom, halogeniertem Alkyl, wie Trifluormethyl, Aryl, wie Phenyl, oder unsubstituierte Arylgruppen.

Die Reste $R^{11}$ und $R^{12}$ können einzeln oder verbrückt sein. Auch die Reste $R^{21}$ und $R^{22}$ können einzeln oder verbrückt sein. Die Reste $R^{11}$, $R^{12}$, $R^{21}$ und $R^{22}$ können alle einzeln, zwei verbrückt und zwei einzeln oder alle vier verbrückt sein in der beschriebenen Art.

In einer besonders bevorzugten Ausführungsform kommen die in US 5,723,641 genannten Verbindungen der Formel I, II, III, IV und V in Betracht. In einer besonders bevorzugten Ausführungsform kommen die in US 5,512,696 genannten Verbindungen der Formel I, II, III IV, V, VI und VII, insbesondere die dort in den Beispielen 1 bis 31 eingesetzten Verbindungen, in Betracht. In einer besonders bevorzugten Ausführungsform kommen die in US 5,821,378 genannten Verbindungen der Formel I, II, III, IV, V, VI, VII, VIII, IX, X, XI, XII, XIII, XIV und XV, insbesondere die dort in den Beispielen 1 bis 73 eingesetzten Verbindungen, in Betracht.

In einer besonders bevorzugten Ausführungsform kommen die in US 5,512,695 genannten Verbindungen der Formel I, II, III, IV, V und VI, insbesondere die dort in den Beispielen 1 bis 6 eingesetzten Verbindungen, in Betracht. In einer besonders bevorzugten Ausführungsform kommen die in US 5,981,772 genannten Verbindungen der Formel I, II, III, IV, V, VI, VII, VIII, IX, X, XI, XII, XIII und XIV, insbesondere die dort in den Beispielen 1 bis 66 eingesetzten Verbindungen, in Betracht.

In einer besonders bevorzugten Ausführungsform kommen die in US 6,127,567 genannten Verbindungen und dort in den Beispielen 1 bis 29 eingesetzten Verbindungen in Betracht. In einer besonders bevorzugten Ausführungsform kommen die in US 6,020,516 genannten Verbindungen der Formel I, II, III, IV, V, VI, VII, VIII, IX und X, insbesondere

die dort in den Beispielen 1 bis 33 eingesetzten Verbindungen, in Betracht. In einer besonders bevorzugten Ausführungsform kommen die in US 5,959,135 genannten Verbindungen und dort in den Beispielen 1 bis 13 eingesetzten Verbindungen in Betracht.

In einer besonders bevorzugten Ausführungsform kommen die in US 5,847,191 genannten Verbindungen der Formel I, II und III in Betracht. In einer besonders bevorzugten Ausführungsform kommen die in US 5,523,453 genannten Verbindungen, insbesondere die dort in Formel 1, 2, 3, 4, 5, 6, 7, 8, 9, 10, 11, 12, 13, 14, 15, 16, 17, 18, 19, 20 und 21 dargestellten Verbindungen, in Betracht. In einer besonders bevorzugten Ausführungsform kommen die in WO 01/14392 genannten Verbindungen, vorzugsweise die dort in Formel V, VI, VII, VIII, IX, X, XI, XII, XIII, XIV, XV, XVI, XVII, XXI, XXII, XXIII dargestellten Verbindungen, in Betracht.

In einer besonders bevorzugten Ausführungsform kommen die in WO 98/27054 genannten Verbindungen in Betracht. In einer besonders bevorzugten Ausführungsform kommen die in WO 99/13983 genannten Verbindungen in Betracht. In einer besonders bevorzugten Ausführungsform kommen die in WO 99/64155 genannten Verbindungen in Betracht.

In einer besonders bevorzugten Ausführungsform kommen die in der deutschen Patentanmeldung DE 100 380 37 genannten Verbindungen in Betracht. In einer besonders bevorzugten Ausführungsform kommen die in der deutschen Patentanmeldung DE 100 460 25 genannten Verbindungen in Betracht. In einer besonders bevorzugten Ausführungsform kommen die in der deutschen Patentanmeldung DE 101 502 85 genannten Verbindungen in Betracht.

In einer besonders bevorzugten Ausführungsform kommen die in der deutschen Patentanmeldung DE 101 502 86 genannten Verbindungen in Betracht. In einer besonders bevorzugten Ausführungsform kommen die in der deutschen Patentanmeldung DE 102 071 65 genannten Verbindungen in Betracht. In einer weiteren besonders bevorzugten Ausführungsform der vorliegenden Erfindung kommen die in der US 2003/0100442 A1 genannten phosphorhaltigen Chelatliganden in Betracht.

In einer weiteren besonders bevorzugten Ausführungsform der vorliegenden Erfindung kommen die in der nicht vorveröffentlichten deutschen Patentanmeldung Aktenzeichen DE 103 50 999.2 vom 30.10.2003 genannten phosphorhaltigen Chelatliganden in Betracht.

Die beschriebenen Verbindungen I, Ia, Ib und II sowie deren Herstellung sind an sich bekannt. Als phosphorhaltiger Ligand können auch Mischungen, enthaltend mindestens zwei der Verbindungen I, Ia, Ib und II, eingesetzt werden.

In einer besonders bevorzugten Ausführungsform des erfindungsgemäßen Verfahrens ist der phosphorhaltige Ligand des Nickel(0)-Komplexes und/oder der freie phosphorhaltige Ligand ausgewählt aus Tritolylphosphit, bidentaten phosphorhaltigen Chelatliganden, sowie den Phosphiten der Formel Ib

$$P(O\text{-}R^1)_x\,(O\text{-}R^2)_y\,(O\text{-}R^3)_z\,(O\text{-}R^4)_p \qquad \text{(Ib)}$$

worin $R^1$, $R^2$ und $R^3$ unabhängig voneinander ausgewählt sind aus o-Isopropyl-phenyl, m-Toiyl und p-Tolyl, $R^4$ Phenyl ist; x gleich 1 oder 2 ist, und y, z, p unabhängig voneinander 0, 1 oder 2 sind mit der Maßgabe, dass x+y+z+p = 3 ist; und deren Mischungen.

Lewis-Säure bzw. Promotor

Im Sinne der vorliegenden Erfindung wird unter einer Lewis-Säure eine einzelne Lewis-Säure, wie auch ein Gemisch aus mehreren, wie zwei, drei oder vier Lewis-Säuren, verstanden.

Als Lewis-Säure kommen dabei anorganische oder organische Metall-Verbindungen in Betracht, in denen das Kation ausgewählt ist aus der Gruppe bestehend aus Scandium, Titan, Vanadium, Chrom, Mangan, Eisen, Kobalt, Kupfer, Zink, Bor, Aluminium, Yttrium, Zirkonium, Niob, Molybdän, Cadmium, Rhenium und Zinn. Beispiele umfassen $ZnBr_2$, $ZnI_2$, $ZnCl_2$, $ZnSO_4$, $CuCl_2$, CuCl, $Cu(O_3SCF_3)_2$, $CoCl_2$, $CoI_2$, $FeI_2$, $FeCl_3$, $FeCl_2$, $FeCl_2(THF)_2$, $TiCl_4(THF)_2$, $TiCl_4$, $TiCl_3$, $ClTi(O\text{-}i\text{-}Propyl)_3$, $MnCl_2$, $ScCl_3$, $AlCl_3$, $(C_8H_{17})AlCl_2$, $(C_8H_{17})_2AlCl$, $(i\text{-}C_4H_9)_2AlCl$, $(C_6H_5)_2AlCl$, $(C_6H_5)AlCl_2$, $ReCl_5$, $ZrCl_4$, $NbCl_5$, $VCl_3$, $CrCl_2$, $MoCl_5$, $YCl_3$, $CdCl_2$, $LaCl_3$, $Er(O_3SCF_3)_3$, $Yb(O_2CCF_3)_3$, $SmCl_3$, $B(C_6H_5)_3$, $TaCl_5$, wie beispielsweise in US 6,127,567, US 6,171,996 und US 6,380,421 beschrieben. Weiterhin kommen in Betracht Metallsalze, wie $ZnCl_2$, $CoI_2$ und $SnCl_2$ und organometallische Verbindungen, wie $RAlCl_2$, $R_2AlCl$, $RSnO_3SCF_3$ und $R_3B$, wobei R eine Alkyl- oder Aryl-Gruppe ist, wie beispielsweise in US 3,496,217, US 3,496,218 und US 4,774,353 beschrieben.

Weiterhin können gemäß US 3,773,809 als Promotor ein Metall in kationischer Form, ausgewählt aus der Gruppe bestehend aus Zink, Cadmium, Beryllium, Aluminium, Gallium, Indium, Thallium, Titan, Zirkonium, Hafnium, Erbium, Germanium, Zinn, Vanadium, Niob, Scandium, Chrom, Molybdän, Wolfram, Mangan, Rhenium, Palladium, Thorium, Eisen und Kobalt, vorzugsweise Zink, Cadmium, Titan, Zinn, Chrom, Eisen und Kobalt, eingesetzt werden, wobei der anionische Teil der Verbindung ausgewählt sein kann aus der Gruppe bestehend aus Halogeniden, wie Fluorid, Chlorid, Bromid und Jodid, Anionen niedriger Fettsäuren mit von 2 bis 7 Kohlenstoffatomen, $HPO_3^{2-}$, $H_3PO^{2-}$,

$CF_3COO^-$, $C_7H_{15}OSO_2^-$ oder $SO_4^{2-}$. Weiterhin sind aus US 3,773,809 als geeignete Promotoren Borhydride, Organoborhydride und Borsäureester der Formel $R_3B$ und $B(OR)_3$, wobei R ausgewählt ist aus der Gruppe bestehend aus Wasserstoff, Aryl-Radikale mit zwischen 6 und 18 Kohlenstoff-Atomen, mit Alkyl-Gruppen mit 1 bis 7 Kohlenstoff-Atomen substituierte Aryl-Radikale und mit Cyano-substituierte Alkyl-Gruppen mit 1 bis 7 Kohlenstoff-Atomen substituierte Aryl-Radikale, vorteilhaft Triphenylbor, genannt.

Weiterhin können, wie in US 4,874,884 beschrieben, synergistisch wirksame Kombinationen von Lewis-Säuren eingesetzt werden, um die Aktivität des Katalysatorsystems zu erhöhen. Geeignete Promotoren können beispielsweise aus der Gruppe bestehend aus $CdCl_2$, $FeCl_2$, $ZnCl_2$, $B(C_6H_5)_3$ und $(C_6H_5)_3SnX$, mit X gleich $CF_3SO_3$, $CH_3C_6H_4SO_3$ oder $(C_6H_5)_3BCN$ ausgewählt werden, wobei für das Verhältnis von Promotor zu Nickel ein Bereich von vorzugsweise etwa 1:16 bis etwa 50:1 genannt ist.

Im Sinne der vorliegenden Erfindung umfasst der Begriff Lewis-Säure auch die in US 3,496,217, US 3,496,218, US 4,774,353, US 4,874,884, US 6,127,567, US 6,171,996 und US 6,380,421 genannten Promotoren.

Als besonders bevorzugte Lewis-Säuren kommen unter den genannten insbesondere Metallsalze, besonders bevorzugt Metallhalogenide, wie Fluoride, Chloride, Bromide, Jodide, insbesondere Chloride, in Betracht, von denen wiederum Zinkchlorid, Eisen-(II)-chlorid und Eisen-(III)-chlorid besonders bevorzugt sind.

Mit dem erfindungsgemäßen Verfahren sind eine Reihe von Vorteilen verbunden. So ist die Hydrocyanierung von 3-Pentennitril mit einem geringen Umsatzgrad möglich, ohne dass zur Ermöglichung der Phasentrennung in der vorgesehenen extraktiven Abtrennung des Katalysatorsystems entweder 3-Pentennitril vorverdampft oder Adipodinitril zur Verdünnung zugegeben werden muss. Die ermöglichte Fahrweise der Hydrocyanierung mit geringerem Umsatzgrad an 3-Pentennitril ist verbunden mit einer besseren Selektivität von Adipodinitril bezüglich 3-Pentennitril und Cyanwasserstoff. Die ermöglichte Fahrweise der Hydrocyanierung mit geringerem Umsatzgrad an 3-Pentennitril ist darüber hinaus mit einer höheren Stabilität des Katalysatorsystems verbunden.

Durch die optionale Behandlung des Reaktionsaustrages mit Ammoniak bzw. Aminen, und die optionale Abtrennung der Feststoffe vom Reaktionsaustrag, lässt sich das Verfahren weiter optimieren, und die Trennleistung der Extraktion einstellen.

Beispiele

Im Folgenden angegebene Prozentzahlen sind Massenprozent bezüglich der Mischung aus Adipodinitril (ADN), 3-Pentennitril (3PN) und dem jeweiligen Liganden. Cyclohexan wurde nicht in die Berechnung einbezogen.

Beispiel I

In einem Glaskolben wurden unter Schutzgasatmosphäre (Argon) 5 g einer Mischung (Zusammensetzung siehe Tabelle) aus ADN, 3PN und Tritolylphosphit (TTP) als Ligand angesetzt und anschließend 5 g Cyclohexan zugegeben. Durch Rühren bei einer definierten Temperatur wurde eine Vermischung der Komponenten erzielt. Nach Abstellen des Rührorgans wurde bei weitergeführter Temperierung visuell die Phasentrennung verfolgt. Wenn nach 5 min. visuell keine zwei getrennten Phasen erkennbar waren, so wurde das System als nicht in separate Phasen getrennt bewertet. Die Ergebnisse sind in Tabelle 1 zusammengestellt.

Tabelle 1:

| ADN | Ligand TTP | 3PN | Phasentrennung 20°C | Phasentrennung 40°C | Phasentrennung 60°C |
|---|---|---|---|---|---|
| 30 % | 0 % | 70 % | nein | nein | nein |
| 20 % | 10 % | 70 % | nein | nein | nein |
| 40 % | 0 % | 60 % | ja | ja | nein |
| 30 % | 10 % | 60 % | ja | nein | nein |
| 20 % | 20 % | 60 % | nein | nein | nein |
| 40 % | 10 % | 50 % | ja | ja | nein |
| 30 % | 20 % | 50 % | ja | nein | nein |
| 50 % | 10 % | 40 % | ja | ja | ja |
| 30 % | 30 % | 40 % | ja | nein | nein |
| 50 % | 20 % | 30 % | ja | ja | ja |

(fortgesetzt)

| ADN | Ligand TTP | 3PN | Phasentrennung 20°C | Phasentrennung 40°C | Phasentrennung 60°C |
|---|---|---|---|---|---|
| 50 % | 30 % | 20 % | ja | ja | ja |
| 60 % | 20 % | 20 % | ja | ja | ja |

Beispiel II

Das Vorgehen entspricht dem in Beispiel I, wobei jedoch ein Chelatligand der Formel A anstelle von Tritolylphosphit verwendet wurde. Die Ergebnisse sind in Tabelle 2 zusammengestellt.

**Formel A:**

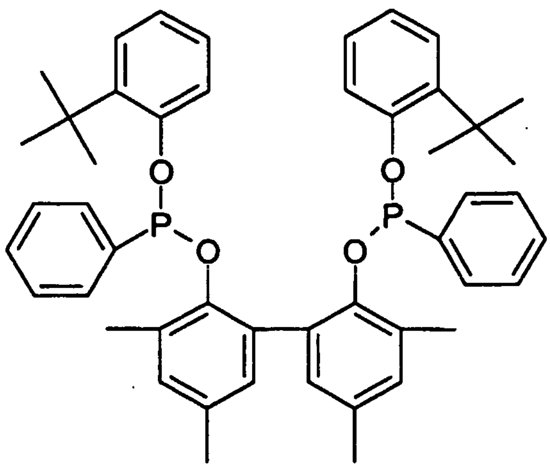

Tabelle 2:

| ADN | Ligand Formel A | 3PN | Phasentrennung 20 °C | Phasentrennung 40 °C | Phasentrennung 60 °C |
|---|---|---|---|---|---|
| 30 % | 0 % | 70 % | ja | ja | nein |
| 20 % | 10 % | 70 % | nein | nein | nein |
| 40 % | 0 % | 60 % | ja | ja | ja |
| 30 % | 10 % | 60 % | ja | nein | nein |
| 20 % | 20 % | 60 % | nein | nein | nein |
| 40 % | 10 % | 50 % | ja | ja | ja |
| 30 % | 20 % | 50 % | ja | ja | nein |
| 50 % | 10 % | 40 % | ja | ja | ja |
| 30 % | 30 % | 40 % | ja | ja | ja |
| 50 % | 20 % | 30 % | ja | ja | ja |
| 50 % | 30 % | 20 % | Ja | ja | ja |
| 60 % | 20 % | 20 % | Ja | ja | ja |

Beispiel III

Das Vorgehen entspricht dem in Beispiel I, wobei jedoch ein Chelatligand der Formel B anstelle von Tritolylphosphit verwendet wurde. Die Ergebnisse sind in Tabelle 3 zusammengestellt.

Tabelle 3:

| ADN | Ligand Formel B | 3PN | Phasentrennung 20 °C | Phasentrennung 40 °C | Phasentrennung 60 °C |
|---|---|---|---|---|---|
| 20 % | 10 % | 70 % | nein | nein | nein |
| 30 % | 10 % | 60 % | ja | ja | nein |
| 30 % | 20 % | 50 % | ja | ja | nein |
| 60 % | 20 % | 20 % | ja | ja | ja |

Formel B:

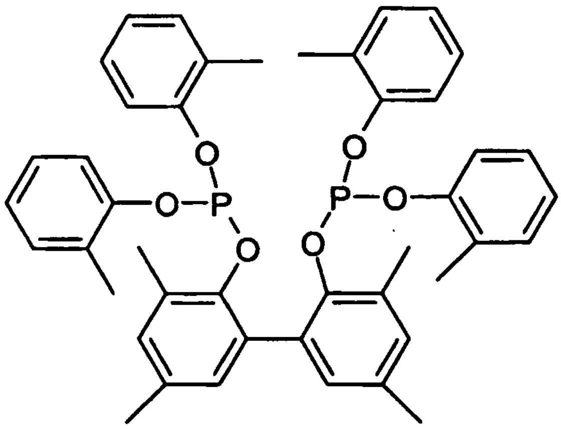

Die folgenden Beispiele IV und V illustrieren die vorteilhafte Wirkung einer Feststoffabtrennung.

Beispiel IV: ohne Feststoffabtrennung

4 Volumenteile einer Mischung aus ADN, 3PN und Chelatligand der Formel A wurden mit einem Volumenteil des Kohlenwasserstoffs extrahiert. Der verwendete Kohlenwasserstoff, die Zusammensetzung der Mischung und die Temperatur bei der Extraktion und der Phasentrennung sind Tabelle 4 zu entnehmen.

Die bei der Extraktion erhaltenen mehrphasigen Gemische wurden bei definierter Temperatur in verschlossen Probengläschen stehen gelassen. Nach einer bestimmten Zeit wurde die Güte der Phasentrennung visuell beurteilt. Tabelle 4 fasst die Ergebnisse zusammen.

Tabelle 4: Phasentrennung

| Zusammens. [Gew.-%] | | Temp. [°C] [2)] | Dauer des Stehenlassens | Phasentrennung bei Verwendung von ▼ als Kohlenwasserstoff | | | |
|---|---|---|---|---|---|---|---|
| ADN/3PN [1)] | Ligand Formel A | | | Cyclohexan | Methylcyclohexan | n-Heptan | n-Octan |
| 65 | 35 | 23 | 10 min | nein | nein | nein | nein |
| 55 | 45 | 40 | 10 min | nein | nein | nein | nein |
| 40 | 60 | 70 | 2 min | nein | nein | Grobtrennung | Grobtrennung |
| 40 | 60 | 70 | 10 min | nein | nein | Grobtrennung | Grobtrennung |
| 40 | 60 | 70 | 3 Tage | Trennung, jedoch viel Mulm | Trennung, jedoch viel Mulm | Trennung, wenig Mulm | Trennung, wenig Mulm |

[1)] Mischung aus 60 Gew.-% ADN und 40 Gew.-% 3PN
[2)] Temperatur bei Extraktion, Phasentrennung und Stehenlassen

Beispiel V: mit Feststoffabtrennung

Beispiel V wurde wiederholt, jedoch wurde der in der Reaktionsmischung enthaltene Feststoff vor der Extraktion in einem Dekanter abgetrennt. Die Phasentrennzeit bis zur . Grobtrennung der Phasen wurde ermittelt Sie ist in Tabelle 5 der Trennzeit von Beispiel IV gegenübergestellt.

Tabelle 5: Phasentrennzeiten [sec] ohne Feststoff (Beispiel V) und mit Feststoff (Beispiel IV) bis zur Grobtrennung; F. bedeutet Feststoff

| Kohlenwass. ► Temperatur ▼ | Cyclohexan | Methylcyclohexan | n-Heptan | n-Octan |
|---|---|---|---|---|
| 23°C | | | | |
| ohne F. | > 600 | > 600 | > 600 | > 600 |
| mit F. | > 600 | > 600 | > 600 | > 600 |
| 40°C | | | | |
| ohne F. | > 600 | > 600 | 150 | 180 |
| mit F. | > 600 | > 600 | > 600 | > 600 |
| 50°C | | | | |
| ohne F. | 180 | 250 | 70 | 70 |
| mit F. | > 600 | > 600 | > 600 | > 600 |
| 70°C | | | | |
| ohne F. | 80 | 80 | 10 | 20 |
| mit F. | 300 | 300 | 60 | 100 |

Demnach waren die Phasentrennzeiten nach einer Abtrennung des Feststoffs kürzer als ohne Feststoffabtrennung.

Die folgenden Beispiele VI bis IX illustrieren die vorteilhafte Wirkung einer Behandlung mit Ammoniak.

Beispiel VI-a: ohne Ammoniakbehandlung

In einer kontinuierlich betriebenen vierstufigen Mixer-Settler-Extraktionsapparatur (ca. 150 ml Volumen pro Mixer und Settler) wurde im Gegenstrom ein Zulauf bei 40 °C mit n-Heptan extrahiert. Der Zulauf enthielt 27,5 Gew.-% Pentennitrile, 27,5 Gew.-% Adipodinitril und 45 Gew.-% Katalysator, wobei der Katalysator den Liganden der Formel A, außerdem Nickel(0) (komplexiert am Liganden vorliegend), und schließlich $ZnCl_2$ enthielt, und das molare Verhältnis dieser drei Katalysatorkomponenten 1: 1 : 1 betrug.

Die erhaltenen Ober- und Unterphasen wurden kontinuierlich destillativ vom Extraktionsmittel befreit, und dieses für die Extraktion rückgeführt. Die Apparatur wurde mit 100 g/h Zulauf und 100 g/h n-Heptan betrieben, bis sich nach 30 Stunden Stationarität einstellte. Danach wurden Zu- und Abflüsse unter denselben Bedingungen für eine Stunde bilanziert.

Die Bilanz wurde erstellt, indem mittels Elementaranalyse der Gehalt an Phosphor (als Maß für den phosphorhaltigen Liganden) und Nickel (als Maß für komplexierte Katalysator-Aktivkomponente) im Zulauf und der erhaltenen gesammelten Ober- bzw. Unterphase bestimmt und ausgewertet wurde. Die Genauigkeit der Bilanzierung betrug $\pm 5$ %, weshalb die Summe der %-Werte von Ober- und Unterphase nicht immer genau 100 % ergibt.

Die Bilanzen der folgenden Beispiele wurden in gleicher Weise erstellt. Tabelle 6 fasst die Bilanzen zusammen.

Biespiel VI-b

Beispiel VI-a wurde wiederholt, wobei das molare Verhältnis der drei Katalysatorkomponenten (Ligand der Formel A, komplexiertes Nickel (0) und $ZnCl_2$) 2:1:1 1 betrug.

Beispiel VII: mit Ammoniakbehandlung, ohne Feststoffabtrennung

Beispiel VI-a wurde wiederholt, jedoch wurde vor der Extraktion der Zulauf in einem 4 l-Rundkolben unter Rühren bei 40 °C mit 2,2 Mol-Äquivalenten (bezogen auf das enthaltene $ZnCl_2$) gasförmigem, trockenem Ammoniak versetzt. Das eingeleitete Ammoniak wurde vollständig von der Lösung aufgenommen. Nach der Einleitung wurde ggf. überschüssiges Ammoniak mittels Durchleiten von Argon entfernt.

Bei der Ammoniakeinleitung fiel ein heller, fein kristalliner Feststoff aus, der im Zulauf verblieb und mit durch die Extraktion gefahren wurde. Der Großteil des Feststoffes wurde mit der Unterphase aus der Extraktionsapparatur ausgetragen; ein kleiner Teil sedimentierte und verblieb in der Extraktionsapparatur.

Beispiel VIII: mit Ammoniakbehandlung, mit Feststoffabtrennung durch Filtration

Beispiel VII wurde wiederholt, jedoch wurde nach dem Einleiten des Ammoniak und vor der Extraktion der ausgefallene Feststoff durch Filtration über eine Druckfilternutsche (Tiefenfilter Fa. Seitz, K 700) abgetrennt.

Beispiel IX: mit Ammoniakbehandlung, mit Feststoffabtrennung durch Dekantieren

Beispiel VII wurde wiederholt; jedoch betrug das molare Verhältnis der drei Katalysatorkomponenten (Ligand der Formel A, komplexiertes Nickel (0) und $ZnCl_2$) 2:1:1, und es wurde nach dem Einleiten des Ammoniak und vor der Extraktion der ausgefallene Feststoff durch Sedimentation und anschließendes Dekantieren abgetrennt.

Tabelle 6: Bilanzierung [%] für Ligand und Nickel(0) (Genauigkeit ±5 %)

| Bilanz [%] ► Beispiel ▼ | Ligand in der Oberphase | Nickel in der Oberphase | Ligand in der Unterphase | Nickel in der Unterphase |
|---|---|---|---|---|
| VI-a | 25 | 28 | 72 | 70 |
| VI-b | 51 | 22 | 53 | 76 |
| VII | 99 | 96 | < 0,1 | < 0,1 |
| VIII | 97 | > 99 | < 0,1 | < 0,1 |
| IX | > 99 | > 99 | <0,1 | <0,1 |

Die Beispiele VI bis IX zeigen, dass sich durch die Ammoniakbehandlung (Beispiele VIII bis IX) die Anreicherung von Ligand und Nickel-Komplex in der Oberphase deutlich verbesserte. Durch die Feststoffabtrennung vor der Extraktion (Beispiele VIII und IX) konnte die Anreicherung nochmals verbessert werden.

## Patentansprüche

1. Verfahren zur extraktiven Abtrennung von Nickel(0)-Komplexen mit phosphorhaltigen Liganden und/oder freien phosphorhaltigen Liganden, aus einem Reaktionsaustrag einer Hydrocyanierung von ungesättigten Mononitrilen zu Dinitrilen, durch Extraktion mittels eines Kohlenwasserstoffs, wobei bei einer Temperatur T (in °C) eine Phasentrennung des Kohlenwasserstoffs und des Reaktionsaustrages in zwei Phasen erfolgt,
**dadurch gekennzeichnet, dass** im Reaktionsaustrag der Hydrocyanierung der Gehalt an Nickel(0)-Komplexen mit phosphorhaltigen Liganden und/oder freien phosphorhaltigen Liganden, abhängig von der Temperatur T mindestens y Gew.-% beträgt, und unabhängig von der Temperatur T maximal 60 Gew.-% beträgt, wobei der Zahlenwert des Mindestgehalts y durch die Gleichung

$$y = 0,5 \cdot T + 20$$

gegeben ist und T in die Gleichung als dimensionsloser Zahlenwert einzusetzen ist.

2. Verfahren nach Anspruch 1, **dadurch gekennzeichnet, dass** man den Reaktionsaustrag der Hydrocyanierung vor oder während der Extraktion mit Ammoniak oder einem primären, sekundären oder tertiären aromatischen oder

aliphatischen Amin behandelt.

3. Verfahren nach den Ansprüchen 1 bis 2, **dadurch gekennzeichnet, dass** man den Reaktionsaustrag mit wasserfreiem Ammoniak behandelt.

4. Verfahren nach den Ansprüchen 1 bis 3, **dadurch gekennzeichnet, dass** man als Kohlenwasserstoff Cyclohexan, Methylcyclohexan, n-Heptan oder n-Octan verwendet.

5. Verfahren nach den Ansprüchen 1 bis 4, **dadurch gekennzeichnet, dass** man als Kohlenwasserstoff n-Heptan oder n-Octan verwendet.

6. Verfahren nach den Ansprüchen 1 bis 5, **dadurch gekennzeichnet, dass** man vor der Extraktion die im Reaktionsaustrag enthaltenen Feststoffe zumindest teilweise abtrennt.

7. Verfahren nach den Ansprüchen 1 bis 6, **dadurch gekennzeichnet, dass** man die Phasentrennung der Extraktion bei einer Temperatur von -15 bis 120°C durchführt.

8. Verfahren nach den Ansprüchen 1 bis 7, **dadurch gekennzeichnet, dass** in demjenigen Bereich der Extraktion, worin der Gehalt an Nickel(0)-Komplexen mit phosphorhaltigen Liganden und/oder freien phosphorhaltigen Liganden höher ist als im anderen Bereich, die Temperatur niedriger ist als im anderen Bereich.

9. Verfahren nach den Ansprüchen 1 bis 8, **dadurch gekennzeichnet, dass** der phosphorhaltige Ligand ausgewählt ist ausgewählt aus mono- oder bidentaten Phosphinen, Phosphiten, Phosphiniten und Phosphoniten.

10. Verfahren nach den Ansprüchen 1 bis 9, **dadurch gekennzeichnet, dass** der phosphorhaltige Ligand ausgewählt ist aus Tritolylphosphit, bidentaten phosphorhaltigen Chelatliganden, sowie Phosphiten der Formel Ib

$$P(O\text{-}R^1)_x(O\text{-}R^2)_y(O\text{-}R^3)_z(O\text{-}R^4)_p \qquad \text{(Ib)}$$

worin $R^1$, $R^2$ und $R^3$ unabhängig voneinander ausgewählt sind aus o-Isopropylphenyl, m-Tolyl und p-Tolyl, $R^4$ Phenyl ist, x gleich 1 oder 2 ist, und y, z, p unabhängig voneinander 0, 1 oder 2 sind, mit der Maßgabe, dass x+y+z+p = 3 ist; und deren Mischungen.

11. Verfahren nach den Ansprüchen 1 bis 10, **dadurch gekennzeichnet, dass** das Mononitril 3-Pentennitril und das Dinitril Adipodinitril ist.

12. Verfahren nach den Ansprüchen 1 bis 11, **dadurch gekennzeichnet, dass** man den Reaktionsaustrag durch Umsetzung von 3-Pentennitril mit Cyanwasserstoff in Gegenwart mindestens eines Nickel(0)-Komplexes mit phosphorhaltigen Liganden, gegebenenfalls in Gegenwart mindestens einer Lewis-Säure, erhält.

**Claims**

1. A process for extractively removing nickel (0) complexes having phosphorus ligands and/or free phosphorus ligands from a reaction effluent of a hydrocyanation of unsaturated mononitriles to dinitriles by extraction by means of a hydrocarbon, a phase separation of the hydrocarbon and of the reaction effluent into two phases being effected at a temperature T (in °C),
wherein the content of nickel (0) complexes having phosphorus ligands and/or free phosphorus ligands in the reaction effluent of the hydrocyanation, depending on the temperature T, is at least y% by weight and, irrespective of the temperature T, is a maximum of 60% by weight, where the numerical value of the minimum content y is given by the equation

$$y = 0.5 \bullet T + 20$$

and T is to be used in the equation as a dimensionless numerical value.

2. The process according to claim 1, wherein the reaction effluent of the hydrocyanation is treated before or during the extraction with ammonia or a primary, secondary or tertiary aromatic or aliphatic amine.

3. The process according to claims 1 to 2, wherein the reaction effluent is treated with anhydrous ammonia.

4. The process according to claims 1 to 3, wherein the hydrocarbon used is cyclohexane, methylcyclohexane, n-heptane or n-octane.

5. The process according to claims 1 to 4, wherein the hydrocarbon used is n-heptane or n-octane.

6. The process according to claims 1 to 5, wherein the solids present in the reaction effluent are at least partly removed before the extraction.

7. The process according to claims 1 to 6, wherein the phase separation of the extraction is carried out at a temperature of from -15 to 120°C.

8. The process according to claims 1 to 7, wherein, in that region of the extraction in which the content of nickel(0) complexes having phosphorus ligands and/or free phosphorus ligands is higher than in the other region, the temperature is lower than in the other region.

9. The process according to claims 1 to 8, wherein the phosphorus ligand is selected from mono- or bidentate phosphines, phosphites, phosphinites and phosphonites.

10. The process according to claims 1 to 9, wherein the phosphorus ligand is selected from tritolyl phosphite, bidentate phosphorus chelate ligands, and phosphites of the formula Ib

$$P(O\text{-}R^1)_x\,(O\text{-}R^2)_y\,(O\text{-}R^3)_z\,(O\text{-}R^4)_p \qquad \text{(Ib)}$$

where $R^1$, $R^2$ and $R^3$ are each independently selected from o-isopropylphenyl, m-tolyl and p-tolyl, $R^4$ is phenyl, x is 1 or 2, and y, z, p are each independently 0, 1 or 2, with the proviso that x+y+z+p = 3; and mixtures thereof.

11. The process according to claims 1 to 10, wherein the mononitrile is 3-pentenenitrile and the dinitrile is adiponitrile.

12. The process according to claims 1 to 11, wherein the reaction effluent is obtained by reacting 3-pentenenitrile with hydrogen cyanide in the presence of at least one nickel(0) complex having phosphorus ligands, if appropriate in the presence of at least one Lewis acid.

## Revendications

1. Procédé de séparation par extraction de complexes de nickel (0) avec des ligands phosphorés et/ou de ligands phosphorés libres à partir d'un effluent réactionnel d'une hydrocyanation de mononitriles insaturés en dinitriles, par extraction au moyen d'un hydrocarbure, une séparation de l'hydrocarbure et de l'effluent réactionnel en deux phases ayant lieu à une température T (en °C),
**caractérisé en ce que**, dans l'effluent réactionnel de l'hydrocyanation, la teneur en complexes de nickel(0) avec des ligands phosphorés et/ou en ligands phosphorés libres est d'au moins y % en poids en fonction de la température T et au maximum de 60 % en poids, indépendamment de la température T, la valeur numérique de la teneur minimale y étant donnée par l'équation :

$$y = 0{,}5 \cdot T + 20$$

et T étant à introduire dans l'équation sous la forme d'une valeur numérique sans dimension.

2. Procédé suivant la revendication 1, **caractérisé en ce qu'**on traite l'effluent réactionnel de l'hydrocyanation, avant ou pendant l'extraction, avec de l'ammoniac ou une amine primaire, secondaire ou tertiaire, aromatique ou aliphatique.

3. Procédé suivant les revendications 1 et 2, **caractérisé en ce qu'**on traite l'effluent réactionnel avec de l'ammoniac anhydre.

4. Procédé suivant les revendications 1 à 3, **caractérisé en ce que**, comme hydrocarbure, on utilise du cyclohexane, du méthylcyclohexane, du n-heptane ou du n-octane.

5. Procédé suivant les revendications 1 à 4, **caractérisé en ce que**, comme hydrocarbure, on utilise du n-heptane ou du n-octane.

6. Procédé suivant les revendications 1 à 5, **caractérisé en ce que**, avant l'extraction, on sépare au moins partiellement les substances solides contenues dans l'effluent réactionnel.

7. Procédé suivant les revendications 1 à 6, **caractérisé en ce qu'**on effectue la séparation de phases de l'extraction à une température de -15 à 120°C.

8. Procédé suivant les revendications 1 à 7, **caractérisé en ce que**, dans la zone de l'extraction dans laquelle la teneur en complexes de nickel(0) avec des ligands phosphorés et/ou en ligands phosphorés libres est plus élevée que dans l'autre zone, la température est plus basse que dans l'autre zone.

9. Procédé suivant les revendications 1 à 8, **caractérisé en ce que** le ligand phosphoré est choisi parmi des phosphonites, phosphinites, phosphites et phosphines monodentates ou bidentates.

10. Procédé suivant les revendications 1 à 9, **caractérisé en ce que** le ligand phosphoré est choisi parmi du tritolylphosphite, des ligands de chélation phosphorés bidentates, ainsi que des phosphites de formule Ib :

$$P(O\text{-}R^1)_x\,(O\text{-}R^2)_y\,(O\text{-}R^3)_z\,(O\text{-}R^4)_p \qquad \text{(Ib)}$$

dans laquelle $R^1$, $R^2$ et $R^3$ sont choisis indépendamment les uns des autres parmi du o-isopropylphényle, du m-tolyle et du p-tolyle, $R^4$ est du phényle, x est égal à 1 ou 2, et y, z, p représentent, indépendamment les uns des autres 0, 1 ou 2, à la condition que x+y+z+p = 3, et leurs mélanges.

11. Procédé suivant les revendications 1 à 10, **caractérisé en ce que** le mononitrile est du 3-pentènenitrile et le dinitrile de l'adipodinitrile.

12. Procédé suivant les revendications 1 à 11, **caractérisé en ce qu'**on obtient l'effluent réactionnel par réaction de 3-pentènenitrile avec de l'acide cyanhydrique en présence d'au moins un complexe de nickel (0) avec des ligands phosphorés, éventuellement en présence d'au moins un acide de Lewis.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 3773809 A
- US 5932772 A
- US 4339395 A
- WO 2004062765 A
- US 5847191 A
- US 4990645 A
- DE 19953058 A
- US 5723641 A
- US 5512696 A
- US 5821378 A
- US 5512695 A
- US 5981772 A
- US 6127567 A
- US 6020516 A
- US 5959135 A
- US 5523453 A
- WO 0114392 A
- WO 9827054 A
- WO 9913983 A
- WO 9964155 A
- DE 10038037
- DE 10046025
- DE 10150285
- DE 10150286
- DE 10207165
- US 20030100442 A1
- DE 10350999
- US 6171996 B
- US 6380421 B
- US 3496217 A
- US 3496218 A
- US 4774353 A
- US 4874884 A